Europäisches Patentamt

European Patent Office

Office européen des brevets

(11) Publication number: 0 212 285
A1

## EUROPEAN PATENT APPLICATION

(21) Application number: 86110000.6

(22) Date of filing: 21.07.86

(51) Int. Cl.4: **C12N 15/00** , C12P 21/00 , C12N 1/20 , //(C12N15/00,C12R1:19)

(30) Priority: 29.07.85 US 759683

(43) Date of publication of application:
04.03.87 Bulletin 87/10

(84) Designated Contracting States:
AT BE CH DE FR GB IT LI LU NL SE

(71) Applicant: Gottlieb, Arthur A.
5915 Pitt Street
New Orleans Louisiana 70115(US)

(72) Inventor: Gottlieb, Arthur A.
5915 Pitt Street
New Orleans Louisiana 70115(US)

(74) Representative: Popp, Eugen, Dr. et al
MEISSNER, BOLTE & PARTNER
Widenmayerstrasse 48 Postfach 86 06 24
D-8000 München 86(DE)

(54) Derivation and use of selected gene products.

(57) A method of obtaining from a first gentic material a product that is enriched in nucleic acid sequences that are contained in said first genetic material and that is impoverished in nucleic acid sequences that are contained in a second genetic material, said method comprising the following steps:

(1) Providing said first genetic material in denatured form;

(2) Providing fragments of denatured nucleic acid of said second genetic material, said fragments being bound to a support;

(3) Bringing into contact with one another, under hybridizing conditions, said denatured first genetic material and said denatured fragments bound to said support; and

(4) Collecting said denatured first genetic material that has not become bound to said denatured fragments bound to said support, whereby said product is provided.

# DERIVATION AND USE OF SELECTED GENE PRODUCTS

This patent application corresponds to U.S. Pat. App. Ser. No. 759,683, filed 29.7.85, and Convention priority is claimed as to such date.

## BACKGROUND OF THE INVENTION

### Field of the Invention

The present invention relates to means of obtaining selected gene products, particularly the gene sequences responsible for production of materials involved in human T suppressor ("T8") cell activity. Obtaining these gene sequences in relatively purified form makes possible their use in cloning to cause genetically engineered manufacture of the foregoing T8 materials, which are therapeutically useful. Variations of the procedure provide gene products for other human cell activities and also one for analogous animal cells, facilitating genetically engineered manufacture of other human and similar veterinary products. The procedure may also be extended to plant and other genetic material.

### Background

One of the inventors has discovered that lymphocyte dialysates contain immunomodulators, which are materials or substances having therapeutic utility, as described in detail in United States Patent No. 4,468,379. The '379 patent discloses a process for extracting such substances and methods of using them. Other processes are disclosed in pending United States Application Ser. No. 643,724 (EPO No. 85 110 104.8, filed 12.8.85). Such extraction processes begin with human or animal lymphocyte dialysates, and are most costly and time consuming than is manufacture of the desired substances by cloning appropriate genetic material into a host bacterium and thereby causing it to become a living manufacturer of the substances.

The present invention concerns development of a process of the latter type, which is a more advantageous way to obtain the desired immunomodulator substances, particularly the "suppressor" material of the '379 patent. Hitherto, there has been no known or obvious method of accomplishing this purpose. One of the obstacles to development of such a process thus far has been the lack of knowledge of where to find the necessary gene products to clone into a host. However, the inventors have now discovered that the human suppressor material of the '379 patent is generated by T8 cells. Another obstacle has been the difficulty of obtaining the necessary relatively pure T8 gene products to insert into the host bacterium selected to be the manufacturer, since no method of doing so has thus far been known to exist.

Cloning itself is known, although the particular cloning process described herein is believed not to be disclosed in any of the prior art. Cloning is described generally, for example, in Okayama and Berg, High-efficiency cloning of full-length cDNA, 2 Mol. Cell. Biol. 161-70 (1982) --hereinafter "Okayama/cDNA," and Gubler and Hoffman, A simple and very efficient method for generating cDNA libraries, 25 Gene 263-69 (1983) --hereinafter "Gubler/cDNA." Other useful references teaching cloning are the book Maniatis, Fritsch, and Sambrook, Molecular Cloning (1982) --hereinafter "Maniatis/Cloning," and the paper Bahl, Marians, Wu, Stawinsky, and Narang, A general method for inserting specific DNA sequences into cloning vehicles, 1 Gene 81-92 (1976). These references, particularly the book, teach alternative cloning techniques that may be used instead of those described hereinafter.

It is also known that double stranded DNA may be separated ("denatured") into two separate complementary strands, and that that such separate complementary strands, or portions thereof, will anneal to one another under appropriate conditions. It is also known that RNA can be used as a template to cause, by "reverse transcription," the synthesis of DNA that is complementary to the RNA template --such complementary DNA hereinafter being termed "cDNA." See, e.g., Okayama/cDNA.

Use of subtractive, "probe" techniques with DNA is known, also. For example, Timberlake, Developmental gene regulation in Aspergillus nidulans, 78 Developmental Biol. 497-510 (1980), describes a DNA hybdridization probe prepared from fungus mycelial cells. The probe was used, in a solution or suspension of unbound genetic materials, to ascertain the number of genes specifically expressed in different phases of fungal development and differentiation for Aspergillus nidulans.

It is also known that a population of DNA molecules may be subjected to cleavage and fractionation, so that strands are cut up into gene sequences. See Goodman et al. U.S. Pat. No. 4,407,948 (1983), Purification of nucleotide sequences suitable for expression in bacteria.

To insure clarity and consistency, it is noted that the following terminology has been used by others and will be used hereinafter. The term cDNA refers to a double stranded DNA, one strand of which is complementary to messenger RNA - (mRNA) and the other strand of which is complementary to the first strand. As a specific example, a sequence or fragment of one strand of a DNA might be "...ACGG..." (i.e., ...adenine-cytosine-guanadine-guanadine...). The complementary strand of the DNA would have the sequence "...TGCC..." (i.e., ...thiamine-guanadine-cytosine-cytosine...). The corresponding sequence in the messenger RNA (mRNA) would be ...UCGG...- (...uracil-cytosine-guanadine-guanadine....), where the mRNA sequence is the same as that of the first strand of DNA except that U (uracil) is substituted for T (thiamine) in the mRNA.

## SUMMARY OF THE PRESENT INVENTION

It is believed that T8 cells and T helper ("T4") cells contain some gene sequences that are the same for both cells, and some gene sequences that are different. (By way of illustration, the gene sequences for T4 cells may be considered to consist of two sets of sequences, set $\underline{A}$ and set $\underline{B}$, so that the whole T4 gene ensemble is $\underline{A+C}$; and the gene sequences for T8 cells may be considered to be $\underline{B+C}$. An alternative model would be one in which both T4 and T8 cells contain gene sequences $\underline{A+B+C}$, but in different proportions, so that the T4 cells have relatively more $\underline{A}$ molecules than $\underline{B}$ molecules while the T8 cells have relatively more $\underline{B}$ molecules than $\underline{A}$ molecules. In a final version of the model, the ratio of the number of $\underline{A}$ molecules to the number of $\underline{B}$ molecules is higher in T4 cells than in T8 cells.)

The gene sequences that are common to both types of cell (the $\underline{C}$ gene sequences referred to above) are believed by the inventors to be associated with activities that are similar in T4 and T8 cells. The different gene sequences (the $\underline{A}$ and $\underline{B}$ above) are believed associated with activities that are not thus similar. One of the activities that the inventors believe is not similar is the suppressor activity described in the '379 patent --that is, the lessening or suppression of the immune response to antigens to which a subject has been exposed. The inventors believe that the genetic material responsible for generating immunomodulator material causing this activity is associated with T8 cells and not with T4 cells, or at least is expressed to a substantially greater extent in T8 cells than in T4 cells.

It is an object of this invention, therefore, to provide a procedure for the extraction of T8-associated genetic material (i.e., the $\underline{B}$ gene sequences of the above illustration). The purpose is to enable genetically engineered manufacture of suppressor material of the '379 patent.

The procedure of this invention begins with extraction, by known procedures, of RNA associated with T4 cells. The T4 RNA is utilized, again by known procedures, to make a complementary DNA, a T4 cDNA. This T4 DNA material (containing $\underline{A+C}$ genetic sequences, in terms of the prior illustration) is denatured, cut up (thereby breaking up large M.W. plasmids containing inserts, which prevents rapid reannealing of plasmids containing the same or different inserts, via their common plasmid sequences), and then bound to an appropriate resin or other support material in an affinity column. T8 RNA ($\underline{B+C}$ above) is extracted by a similar procedure, denatured, and passed through the affinity column. This is a procedure for extraction of genetic material believed by the inventors not to be disclosed or known in the prior art.

The denatured T8 RNA material is thereby separated into two parts. One part ($\underline{C}$ above) is complementary to part of the T4 cDNA that has been bound to the resin of the affinity column (its $\underline{C}$ part) and, because both are single stranded, they have an affinity toward and anneal to one another, and stay bound to the resin or other support material in the affinity column. This part of the T8 genetic material has the gene sequences that the inventors believe to be similar in T4 and T8 cells - (the $\underline{C}$ part in the illustration above); it is therefore not the material of principal interest to the inventors. The other part of the T8 RNA material (its $\underline{B}$ part) does not anneal to the T4 cDNA in the column, because it is not complementary to it. This is the material of principal interest to the inventors, because it contains the dissimilar gene sequences. The latter gene sequences can be used in preparation for a cloning that will insert into a host the T4-dissimilar sequences of T8 DNA. Those are the gene sequences that the inventors consider to be associated with (among other things) production of material having the suppressor activity described in the '379 patent.

The RNA material that passes through the affinity column is therefore collected and is then used to make DNA complementary to the RNA of interest. This cDNA, when inserted into $\underline{E.\ coli,}$ causes the manufacture of the desired suppressor materials, in accordance with the objectives of the invention.

The same procedure is extendable to the production of similar veterinary material and other human material.

## DETAILED DESCRIPTION OF PREFERRED EMBODIMENT

The procedure of this invention provides a means of extracting genetic material from lymphocyte preparations, so that it may be used for cloning and genetically engineered manufacture of biologically active material that is associated with - (i.e., whose in vivo production is controlled by) the extracted genetic material. The procedure for T8 gene sequences has the following principal parts:

(1) T4 RNA and T8 RNA are prepared from lymphocyte extracts (the T4 and T8 RNA being associ ated respectively with T4 and T8 cells), and a T4 cDNA is prepared from the T4 RNA;

(2) the T8 RNA is separated by a differential binding technique into two parts, one of which contains the gene sequences considered to be associated with the T8 cell activities that are not shared with T4 cells, and the other of which parts contains the gene sequences considered to be common to both T4 and T8 activities;

(3) the selected T8 RNA moiety (the one containing the non-T4 gene sequences) is used to manufacture DNA, which is inserted into E. coli and used to manufacture a suppressor-rich material, from which substantially purified suppressor material may be extracted by the means described in the '379 patent or '724 application.

### I. Preparation of T4 and T8 RNA

### RNA extraction

The initial step in the inventors° process is to extract T4 RNA and T8 RNA from sorted lymphocyte extracts (which contain T4 and T8 cells). The sorting for T4 or T8 is expediently accomplished by means of a Becton-Dickinson Model 440 Fluorescence-Activated Cell Sorter. The extraction is expediently done by the method of Chirgwin, Przybyla, MacDonald, and Rutter, Isolation of biologically active ribonucleic acid from sources enriched in ribonuclease , 18 Biochem. 5294-99 - (1979) --hereinafter "Chirgwin/RNA."

The resulting T4 or T8 cell homogenate· is applied to a CsCl cushion as described in Chirgwin/RNA. It is centrifuged at 35,000 RPM for 17 hours at room temperature in a Beckman SW56 rotor. The resulting precipitated RNA is collected in a solution of guanidine HCl and is ethanol-precipitated as described in Chirgwin/RNA.

Poly A+ RNA, or "messenger RNA" ("mRNA"), is selected from the precipitate by means of oligo dT chromatography, as described in Edmonds, Vaughn, and Nakazato, Polyadenylic acid se quences in the heterogenous nuclear RNA and rapidly labeled polyribosomal RNA of Hela Cells, 68 Proc. Nat. Acad. Sci. 1366-40 (1971). The resulting product is a mixture of mRNA materials associated with T4 or T8 cells (whichever one was initially sorted for).

### mRNA cloning

T4 mRNA is cloned from the poly A+ RNA prepared above, by means of the method of Okayama/cDNA or the method of Gubler/cDNA, or any other convenient method. The optimal choice of methods depends on the availability of RNA, since the Gubler/cDNA method, for example, is cheaper and faster, but it requires use of more RNA than does Okayama/cDNA.

Both methods result in the insertion of T4 mRNA into derivatives of plasmid pBR 322 and result in a large number of plasmid-containing colonies on agar plates. The plasmids contain T4 cDNA, where the cDNA has a first strand with a sequence corresponding to that of the mRNA, and there is annealed to the first strand a second strand, which has a sequence that is complementary to the mRNA sequence. Alternative cloning methods known to those skilled in the art may be substituted for those mentioned above.

### Growth of Recombinant Plasmid DNA

A random sampling of the colonies of T4 mRNA is made and the plasmid-containing material is purified, using the method of Birnboim and Dolly, A rapid alkaline extraction procedure for screening recombinant plasmid DNA, 7 Nucleic Acids Res. 1513-23 (1979) --hereinafter "Birnboim/RPDNA."

The purified plasmids are cut with an appropriate restriction enzyme, such as Eco RI - (Bethesda Research Laboratory, Gaitherburg, Md.), and their size is determined by agarose gel electrophoresis with molecular weight standards, to make sure that the cloned inserts are of appropriate size (i.e., approximately 500 to 5000 base pairs). A procedure for thus assaying inserts is described in a late 1984 publication of the inventOrs. See Young and Gottlieb, Screening of M13 mp bacteriophage clones for small inserts by primer extension and insert excis ion, 1 Gene Anal. Techn. 104-08 (1984) --hereinafter "Young/Screening."

(The abbreviation "u" is used hereinafter to represent "micro," so that "ug" means "microgram," and "ul" means "microliter.")

The size of inserts cloned by the method of Okayama/cDNA is determined by excision of insert DNA with restriction enzymes Pst I and Pvu II (New England Biolabs, Beverly, Mass.). The following reaction buffer is used:

60 mM NaCl,

6 mM Tris-HCl pH 7.5,

6 mM MgCl₂,

6 mM 2-mercaptoethanol,

100 ug/ml bovine serum albumin.

1 to 2 ug of plasmid DNA is combined with 25 ul of the reaction buffer. 1 unit of PstI and PvuII per ug of DNA is added. The mixture is incubated at 37°C for 1 hour. It is then applied to an agarose gel and electrophoresis is carried out. The gel is then strained with 5 ug/ml ethidium bromide and it is viewed under ultraviolet light. The migration of the inserts is compared with that of molecular weight standards. The two closest molecular weight standards (bracketing the inserts) may be used to interpolate for the molecular weight of the inserts.

Approximately 15,000 to 20,000 plasmid-containing clones are pooled by inoculation into L-broth medium. It is desirable to use that large a sample to insure a good representation of the different mRNA species present in the lymphocytes used. The rationale of the sample size is based on Hedrick, Cohen, Nielsen, and Davis, Isolation of cDNA clones encoding T cell-specific membrane-associated proteins, 308 Nature 149-52 (1984), which states that approximately 13,000 different mRNA species are present in T cells. Instead of pooling by inoculation into L-broth, the clones may be pooled by washing the surfaces of plates containing the colonies, as follows:

Example 1 --Culturing Plasmids

L-broth medium is added to the surfaces of a set of plates of plasmid-containing colonies. The surface of the plates is lightly washed with the medium. The washes are combined and brought to approximately 10 ml either by adding L-broth medium or spinning down the cells and replacing the supernatant with 10 ml of medium. The fluid is shaken vigorously.

The resulting suspension is put into a 250 ml Erlenmeyer flask and shaken overnight at 37°C. Then, 0.1 ml of the fluid is used to inoculate 25 ml of L-broth medium in a 100 ml flask containing 50 micrograms/ml of ampicillin. The flask is incubated at 37°C with vigorous shaking until the Absorbance in a spectrophotometer at 600 nm is approximately 0.6.

A 200 ml flask, with 500 ml of L broth prewarmed to 37°C, is inoculated with 25 ml of the culture from the 100 ml flask, and is incubated about 2.5 hours at 37°C with shaking, until the Absorbance at 600 nm is approximately 0.4. To this is added 2.5 ml of a solution of chloramphenicol (34 mg/ml in ethanol), thereby providing 160 ug/ml of chloramphenicol in the flask. This is then incubated for 12-16 hours at 37°C with shaking. (The procedure is an adaptation of the method of Maniatis/Cloning.)

The plasmids are then obtained from the bacteria of the culture by means of the method of Birnboim/RPDNA. The culture is spun down at 4200 RPM in a GSA rotor at 4°C for 10 minutes, thereby producing a bacterial pellet, which is set aside. The pellet should be used as soon as possible.

Extraction of Plasmids from Bacteria

Solutions A, B, and C are prepared as follows:

Solution A: To 100 ml water add 0.9 g of glucose, 2.5 ml of 1 M Tris-HCl pH 8.0, and 10 ml of 0.1 M EDTA, thereby making a solution that is 50 mM glucose, 25 mM Tris-HCl, and 10 mM EDTA.

Solution B: 1% SDS in 0.2 M NaOH.

Solution C: To 60 ml of 5 M K acetate add 11.5 ml of glacial acetic acid and 28.5 ml water, thereby making a solution that is 3 M as to K and 5 M as to acetate, with pH 4.8.

Example 2 --Preparation of Purified Plasmids

To 10 ml of Solution A is added 50 ug of lysozyme (Worthington Corp., Malvern, Pa.), and then the bacterial pellet of Example 1. The pellet is resuspended in the solution by vigorous shaking.

The suspension is placed in a 30 ml Corex centrifuge tube and incubated at room temperature for 5 minutes. The culture is divided into two 50 ml tubes.

To each tube is added 20 ml of fresh Solution B, and the tops are covered with parafilm. The tubes are gently inverted several times, and then are let stand on ice for 10 minutes.

To each tube is added 15 ml ice cold Solution C, and the tops are covered with parafilm. The tubes are mixed by inverting tubes sharply several times. The tubes are let stand on ice for 10 minutes.

The tubes are centrifuged at 4°C for 20 minutes at 12,000 G, 9000 RPM in Sorvall HB4 rotor. The supernatant is poured into two new tubes through wet gauze, thereby filtering material at top of tubes.

There is added 0.6 volume of isopropanol to each tube and the mixture is allowed to stand at room temperature for 15 minutes. The tubes are centrifuged at room temperature for 30 minutes at 12,000 G, 9000 RPM in HB4 rotor.

The supernatant is poured off and the pellet is drained. The pellet is dissolved in 4 ml of 10 mM Tris-HCl pH 7.8, 0.1 mM EDTA, thereby preparing a pellet solution.

About 15 ul of the pellet solution is run on a 1% agarose gel to establish whether plasmid DNA is present. If it is not, it is necessary to repeat whole procedure until gel test shows presence of plasmids.

The plasmid samples are purifed on CsCl gradients using the method of Birnboim/RPDNA. The resulting product is a purified plasmid DNA solution.

## II. Separation of T4/T8 DNA/RNA Sequences

The plasmid DNA is then cut up, denatured, and bound to a cellulose matrix to produce a DNA column, by means of a method based on that of Moss, Moore, and Chan, A simple efficient method for coupling DNA to cellulose, 256 J. Biol. Chem. 12655-58 (1981). While the inventors prefer to use a cellulose resin, other support material may be used instead, such as microspheres or silica. Prior to preparation of the column, the purified plasmid DNA solution is fragmented, as described below, to retard self-annealing of the DNA.

Solution D is prepared by adding 4 mg/ml NaBH₄ to 0.6 N NaOH solution. Solution E is a solution of 0.3 M NaCl, 10 mM Tris-HCl pH 7.5, 0.1 mM EDTA.

Example 3 --Cutting up DNA

To 1.5 mg of purified plasmid DNA solution of Example 2 is added 0.75 ml of the following buffer:

100 mM Tris-HCl pH 7.5,

50 mM NaCl,

5 mM MgCl₂,

100 ug/ml bovine serum albumin.

To this is added 1500 units of Eco RI (Bethesda Research Laboratories). The mixture is incubated at 37°C for 1 hour and then the solution is adjusted to 150 mM NaCl and 6 mM 2-mercaptoethanol by adding NaCl and 2-mercaptoethanol. Then 800 units of restriction enzyme Nde I (Bethesda Research Laboratories) is added and the mixture is incubated for 2 hours at 37°C. The solution is set aside.

Phenol is saturated with 1 M Tris base pH 7.8, and then is neutralized with 0.01 M Tris base 7.8 pH. A volume of the neutralized phenol equal to that of the solution set aside is added to the solution and vortexed to mix. The mixture is centrifuged and the phenol layer is discarded. The remainder is ether-extracted twice with water-saturated ether. The ether phase is discarded.

The DNA is then ethanol-precipitated by adding 0.1 volume of 3 mM Na Acetate pH 5.5 and 2 volumes of ethanol. The mixture is placed at -20°C for at least 2 hours and is then centrifuged at 12,000 G. The supernatant is discarded and 1 ml of 95% ethanol is added to the pellet, which can be stored at -20°C.

The DNA may be used by pouring off the ethanol, drying the pellet, and resuspending the pellet in 10 mM Tris pH 7.8, 0.1 mM EDTA. DNA losses in this process are minimal.

Example 4 --Preparation of Activated Dry Cellulose Powder

1 g cellulose powder (Cellex 410, Bio-Rad) is washed by suction with solution of 200 ml of 47.5% ethanol, 20 ml 0.6 N NaOH, thereby removing residual pyridine contaminants from the cellulose. The washed cellulose is placed in a 10-25 ml flask. To the flask is added 2.5 ml 1,4-butanediol diglycidyl ether (Eastman Kodak) and 2.5 ml Solution D.

The flask is stoppered and stirred or rotated at room temperature for 12 hours. Then, 0.5 ml 1,4-butanediol diglycidyl ether and 0.5 ml Solution D are added. The flask is restoppered and stirred or rotated at room temperature for 6 more hours.

The cellulose is suction washed with 250 ml water or until neutral pH is obtained. Then, it is washed quickly with 50 ml 95% ethanol to remove any residual ether. Then it is washed with 100 ml water and then 20 ml 0.1 N NaOH. The activated cellulose resin is stored at 4°C until use. It has been found that up to 2 weeks of storage does not lead to inactivation of this resin.

The cut up T4 cDNA material is next bound to the cellulose resin. A large excess of cDNA is used to exhaust the active sites on the cellulose resin, so that the resin as such will not take up other materials, such as T8 RNA. The purpose is to permit that particular genetic material that is not uniquely associated with T8 cells and is, rather, associated with both T4 and T8 cells (the $\underline{C}$ gene sequences of the illustration given above at the beginning of the "Summary of the Present Invention") to be withdrawn, in whole or at least in large part, from a mixture of: (a) such both T4-and T8-associated RNA material (the $\underline{C}$ sequences of the above illustration) and (b) only T8-associated RNA material (the $\underline{B}$ sequences of the above illustration). This mixture is located in a T8 genetic sample $(\underline{B}+\underline{C}$ above). That withdrawal occurs because the RNA material common to T4 and T8 cells ($\underline{C}$ above) will become bound to the T4 cDNA material (its $\underline{C}$ part) that is bound in the cellulose. The material not withdrawn from the mixture will be the T8 RNA material ($\underline{B}$ above), which is the material of interest. To insure a thorough extraction, the T4 cDNA:T8 RNA ratio should be approximately 100:1 or greater.

## Example 5 --Coupling DNA to Cellulose

300 ug of cut up T4 cDNA of Example 3 is heated at 100°C for 10 minutes, thereby denaturing it. The denatured (single strand) DNA is cooled quickly in water to approximately room temperature, and is then used immediately.

50 mg of activated cellulose resin of Example 4 is added to 100 ul of 0.1 N NaOH solution, in 1.5 ml Eppendorf microfuge tube. It is shaken. The cooled denatured DNA is added to the tube, which is then shaken. The volume in the tube is brought to 220 ul by adding further 0.1 N NaOH solution. The tube is shaken, producing a slurry.

The slurry is pipetted onto a standard microscope slide and spread over half surface area. The slide is left to sit at 21°C in 100% humidity for 4 to 8 hours, and then allowed to dry at room temperature in air for 2 hours.

The concentrated slurry is scraped off the slide with a razor blade, into 1.5 ml Eppendorf microfuge tube. Then it is mixed with 1 ml water by intermittent vortexing.

Low speed centrifugation is used to separate DNA-cellulose material from supernatant. The DNA-cellulose is washed thoroughly with water, using a small sintered glass filter. Then 1 ml of 2 mM ethanolamine in 50 mM $NaBO_4$, pH 8.5, is reacted with the DNA-cellulose for 1 hour, thereby inactivating any unreacted oxirane groups.

A wash with water follows. The resulting DNA-cellulose product yield is about 300 ug of single stranded cut up T4 cDNA bound to about 50 mg - (dry weight) of activated cellulose.

The procedure must be repeated or done in parallel another 4 times to yield a total of 1.2 mg of single stranded cut up T4 cDNA bound to 200 mg of activated cellulose, which is the amount needed for the next step.

## Example 6 --Use of Column

A slurry of 200 mg of T4 cDNA-cellulose product of Example 5 in approximately an equal volume of Solution E, at 72°C, is prepared. Then, 2 ug of T8 mRNA, purified as described above (Part I), is placed in less than 50 ul of water, and is added to the slurry. The slurry is incubated for 4 hours at 72°C.

The slurry is then poured in a 1 ml plastic syringe, plugged with sterile glass wool, thereby providing an affinity column. The RNA wash-through is then collected. The column is washed with three volumes of Solution E at 72°C and the wash-through is collected. The hybridization procedure involving the use of Solution E is one of several hybridization procedures that might be used.

The RNA wash-throughs are combined and concentrated down to about 20 ul by vacuum dialysis in a collodion bag against 5 mM Tris pH 7.5, at 0°C. The RNA wash-through is then collected. The column is washed with three volumes of Solution E at 72°C and the wash-through is collected.

The RNA wash-throughs are combined and concentrated down to about 20 ul by vacuum dialysis in a collodion bag (Schleicher and Schuell) against 5 mM Tris pH 7.5, at 0°C. That is, the wash-throughs are concentrated to 1-2%.

The RNA content of the concentrate is assayed by the method of Maniatis/Cloning. If the DNA content is less than 60 ng, this part of the procedure terminates. This reflects a target yield of approximately 2%. (In that case, the step of the following paragraph is omitted.)

If the DNA content of the wash-through exceeds 60 ng, probably not enough undesired material was removed in the affinity column, or some of the T4 cDNA became unbound. Therefore, the column needs to be washed using conditions that will clean it of bound mRNA. The column is washed with 10 mM Tris-HCl pH 7.5 at a temperature above 90°C, or at room temperature with 0.1 N NaOH. The wash is discarded. The column is washed with Solution E, which is then discarded, and then the material that previously came through the column is reapplied to the column. This material is reapplied to the washed column until there is 60 ng or less of unbound RNA.

The amount of RNA bound by the column may be increased by increasing the NaCl concentration in Solution E. The amount of RNA bound by the column may be decreased by decreasing the NaCl concentration in Solution E. The RNA assay is repeated to ascertain whether DNA content is less than 60 ng. This procedure is repeated until the 2% target yield is realized (or the procedure is aborted because of repeated failures). After removal of the necessary amount of RNA, the sample is concentrated by vacuum-dialysis in a collodion bag (Schleicher & Schuell).

Then, about 1/3 volume ethanol is added. The mRNA product is then stored at -20°C in a microfuge tube. The sample is dried under nitrogen and may subsequently be reconstituted in water or buffer.

The product of this procedure is a mixture of substantially only T8-associated mRNA, and only very small amounts of T4/T8-associated mRNA and extraneous materials.

The result of this procedure has been to bind the single-stranded T8 RNA material that is common to both T4 and T8 cells to the single-stranded T4 cDNA material that is bound to the cellulose in the affinity column. The T8 RNA material that flows through the affinity column (the wash-throughs) has been greatly (in an efficient, successful run, almost entirely) depleted of its content of the RNA material common to T4 and T8 cells, and has been greatly enriched, percentagewise, in the RNA material that is not common to T4 and T8 cells, i.e., the uniquely T8-associated RNA gene sequence material. The result has been to yield a substantially pure T8 mRNA product, which is substantially free from other genetic material common to T4 and T8 cells.

III. Clonings

The T8 mRNA product of Example 6 is reconstituted with 10 mM Tris, 0.1 mM EDTA, is used to synthesize cDNA, and the cDNA is then cloned by the method of Okayama/cDNA. However, 20 ng of mRNA material suffices, rather than the 200 ng used in Okayama/cDNA, because less than 10 percent of the clones that 200 ng produces are needed here. The resulting clones are then subcloned into the vector. pKK 223-3 (P.L. Biochemicals), to obtain a suitable expression vector. It is not believed that the Okayama/cDNA product is directly usable to give expression of an insert to give a protein product.

It may be found advantageous to substitute other methods for the Okayama/cDNA method. If it is found necessary or desirable to produce a gene product in a mammalian host, rather than a bacterial host, this can be done by the method described in Okayama and Berg, A cDNA cloning vector that permits expression of cDNA inserts in mammalian cells, 3 Molecular and Cellular Biol. 280-89 (1983). This method is similar to that of Okayama/cDNA, but it results in a plasmid that can be grown in E. coli, purified, and transformed into mammalian cells that can be grown in cell culture. This procedure would eliminate the need for the subcloning procedure of Example 7 and might allow production of gene products that cannot be produced in bacteria. A drawback to this method, however, is that culturing mammalian cells is more complex and expensive than is culturing bacteria.

Example 7 --Subcloning mRNA

Procedure A -cutting vector

The pKK 223-3 vector is cut by adding to 5 ug thereof 25 ul of the following buffer:

100 mM Tris-HCl pH 7.5,

5 mM MgCl$_2$,

50 mM NaCl,

100 ug/ml bovine serum albumin.

Then, 5 units of Eco RI is added and the mixture is incubated for 1 hour at 37°C.

The overlapping ends of the cut DNA must be eliminated to give blunt ends suitable for ligation. Therefore, 15 units of T4 DNA polymerase (New England Biolabs) is combined (per 0°Farrell, Replacement synthesis method of labeling DNA fragments, 3(3) Focus 1 (1981)) with 1 ug of plasmid DNA in 25 ul of a buffer consisting of:

16.6 mM (NH$_4$)$_2$SO$_4$,

67 mM Tris-HCl pH 8.8,

6.7 mM MgCl$_2$,

10 mM 2-Mercaptoethanol,

6.7 mM EDTA,

33 uM dATP, dGTP, dCTP, dTTP,

167 ug/ml bovine serum albumin.

The mixture is incubated for 2 hours at 15°C. Then the DNA is ethanol precipated as in Example 3. The DNA precipitate is dried and used in the next step. This procedure may be scaled up as needed.

The precipitate is placed in a 1.5 ml tube. Enough 10 mM Tris pH 7.8 is added to bring the volume to 10 ul/ug of DNA --which is approximately 10 ul. A solution of bacterial alkaline phosphatase - ("BAP"), approximately 50 units/ul, is added to contribute 50 units of BAP/ug DNA --which is approximately 1 ul final volume.

The BAP/DNA mixture is incubated at 65°C for 1 hour. A solution of phenol saturated with 1 M Tris pH 7.8 is prepared, as described in Example 3. The BAP/DNA mixture is added to 10 ul of the phenol solution, vortexed, and centrifuged. The phenol phase is discarded. The phenol extraction is re peated. The DNA is then ethanol-precipitated from the aqueous phase as in Example 3.

The DNA precipitate is dried and then resuspended in enough 10 mM Tris pH 7.8, 0.1 mM EDTA (approximately 10 ul) to provide approximately 0.1 ug DNA/ul of solution. The suspension is frozen and stored at -20°C, until the next step is performed.

Procedure B -Preparation of cDNA from mRNA

The mRNA associated with T8 cells which is the product of Example 6 is used to synthesize a corresponding cDNA, by the method of Okayama/cDNA, using reverse transcriptase as described in Okayama/cDNA. The resulting cDNA product is then used in Procedure C, which follows.

Procedure C -Subcloning

The cDNA of Procedure B is used to prepare plasmids with cloned T8 cDNA inserts, as described in Examples 1 and 2 for culturing plasmids. The plasmid samples from each clone are assayed for size, by agarose gel electrophoresis with molecular weight standards. The size of inserts of interest is approximately 500-5000 bases.

The inserts are excised from the clones having plasmids of approximately the appropriate size, using Pst I and Pvu II. The amount of plasmid DNA used to obtain insert DNA depends on the availability of the plasmid DNA and the length of the insert to be excised. 5-10 ug of plasmid provides adequate amounts of insert in most cases.

The plasmid DNA is added to 25 ul of the following buffer:

60 mM NaCl,

6M Tris-HCl pH 7.5,

6 mM MgCl$_2$,

6 mM 2-mercaptoethanol,

100 ug/ml bovine serum albumin.

To the mixture is added 1 unit of Pvu II per ug of DNA. The mixture is then incubated for 1 hour at 37°C. The mixture is adjusted to 100 mM NaCl, one unit of Pst I per ug of DNA is added, and incubation is continued for one hour at 37°C.

The mixture is applied to a 1% agarose gel and electrophoresis is carried out to separate the smaller insert DNA from the vector DNA. The purified insert DNA is obtained from the gel matrix by the method of Maniatis/Cloning.

The insert DNA is removed from the gel buffer by ethanol precipitation, as described in Example 3. The precipitated DNA is made blunt-ended by use of T4 DNA polymerase. Two units of T4 polymerase is added to 60 ng of insert DNA (DNA concentration determined by the method of Maniatis/Cloning) in 20 ul of the T4 DNA polymerase buffer described in Example 7, Procedure A. The mixture is incubated for 2 hours at 15°C, and then is phenol-extracted with 10 ul of phenol prepared as described in Example 3. The phenol is discarded and the aqueous phase is extracted with water-saturated ether.

The insert DNA is ethanol-precipitated by adding 0.1 volume of 3 M Na Acetate pH 5.6 and 2 volumes of ethanol. The solution is placed at -20°C for at least 3 hours and is then centrifuged at

2.000 G for 10 minutes to precipitate the DNA. The pellet is washed with 95% ethanol, dried, and resuspended in approximately 10 ul of 10 mM Tris pH 7.8, 0.1 mM EDTA.

The DNA concentration is determined by the method of Maniatis/Cloning, using ethidium bromide fluorescence and comparison with standards. The material is ready for insertion into pKK 223-3. It may be stored at -20°C until ready for use.

### Example 8 --DNA Insertion into pKK 233-3

A small amount (less than 1 ml is needed) of Ligation Buffer is prepared --an aqueous solution of 50 mM Tris-HCl pH 7.8, 10 mM $MgCl_2$, 20 mM ATP, and 50 ug/ml BSA.

A ligation mixture, approximately 25 ul, is prepared with the following ingredients:

40 ng pKK 223-3 Eco RI DNA to be cloned (blunt ended and BAP treated).

13 ng of insert DNA pellet,

0.6 unit bacteriophage T4 DNA ligase (Biolabs), and

enough Ligation Buffer to bring volume to 25 ul.

The ligation mixture is incubated overnight at 16°C.

This plasmid material is transformed into E. coli JM 105 by means of the method of Mandel and Higa, Calcium dependent bacteriophage DNA infection 53 J. Mol. Biol. 159-62 (1970).

The resulting clones are set aside for the next step of the procedure. Other cloning procedures kmown to those skilled in the art may be substituted for this one.

### Example 9 --Purification of Cloned Material

The E. coli clones of Example 7 are placed in 2XYT medium, which is as follows:

1.6% (w/v) Bactotryptone (Difco Labs),

1.0% (w/v) Yeast Extract (Difco Labs),

1.0% (w/v) NaCl.

IPTG (isopropylthiogalactoside) is added to this mixture, to a final concentration of 3 mM.

The cells are lysed by using a French Press.

The products manufactured by the clones are separated from the bacterial material by any suitable means, such as ion exchange chromatography, HPLC, gel filtration, electrophoresis, or isoelectric focusing.

The products are then extracted and purified by the method of Gottlieb U.S. Pat. No. 4,468,379 or the method of now pending Gottlieb U.S. Ser. No. 643.379, or a combination thereof. Purified "L-suppressor" material and "S-suppressor" material are extracted separately and assayed for suppressor activity by the method described in the °379 patent; and after confirmation of activity they are set aside and stored for future use.

The procedures described above in several places utilize ethanol-precipitation of DNA. An alternative procedure may be utilized instead, use of the NENSORB₂₀ column (New England Nuclear). In some applications, this procedure is preferable to that described hereinabove. For example, the ability of this system to concentrate and desalt dilute solutions of DNA may facilitate use of the Gubler-Hoffman method for cloning instead of the more time-consuming Okayama/cDNA method. Also, it may facilitate recovery of T4 mRNA from the eluate of the cDNA affinity column of Example 6, replacing vacuum dialysis. However, it may still be necessary to use vacuum dialysis if hybridization solutions other than that mentioned in Example 6 are used. The NENSORB₂₀ column may provide a convenient alternative to phenol extraction for separation of nucleic acids from protein, as in Example 7, Procedure C (subcloning).

It appears that as little as 1 ng of nucleic acid can be purified from any volume of buffer that can be run over the column. A usual procedure for use of the NENSORB₂₀ column is to add to the sample approximately one volume of a Loading Buffer. The Loading Buffer is 0.1 M Tris-HCl pH 7.7, 0.01 M Triethylamine, 1 mM EDTA. The mixture is then loaded on the column. The column is washed with water to remove salts. Then the nucleic acid is eluted from the column with a 50% ethanol in water solution (less than approximately 1 ml). The eluate is then lyophilized, leaving the nucleic acid, which can be placed in an appropriate buffer.

### IV. Related Procedures

The foregoing procedures may be utilized to develop other genetic materials of interest, in humans and animals. The procedure, as taught above, contemplates that there is a first genetic material (e.g. T8 RNA) containing some gene sequences of principal interest, and also containing some other gene sequences that are not of principal interest; and the problem is how to extract the gene sequences of principal interest, or how to separate them from the gene sequences not of principal interest. The procedure of the invention utilizes a second genetic material (e.g., T4 RNA) that also contains the gene sequences not of principal interest, and the procedure provides a way to subtract the latter from the first genetic material.

The second genetic material is made available and used to generate additional complementary genetic material (e.g., T4 cDNA). The inventors have described a method based on the use of reverse transcriptase. However, the invention is more general, in that any suitable method of obtaining complementary genetic material is appropriate, such as synthesizing it. The point is to obtain a complementary genetic material, and the means is inessential. An affinity column is then prepared, to which is bound the cut up, single-stranded form of the genetic material complementary to the second genetic material.

The first genetic material (the mixture containing the gene sequences of interest and also the gene sequences not of interest) is then denatured and passed through the affinity column. (More generally, the genetic materials are brought into contact with one another.) This results in part of the denatured first genetic material (the moiety with the gene sequences not of principal interest) selectively binding to parts of the cut up, denatured material bound in the column, thereby forming double stranded material.

As taught above, the moiety of the first genetic material that is left in the column is that not of principal interest. The wash-through moiety of the first genetic material is the material of principal interest; it was not selected out on the affinity column, because (unlike the material not of interest) it had no affinity for the material bound to the column. The wash-through is therefore collected and used for cloning and manufacture of biologically active nongenetic material (e.g., suppressors) whose production is associated with the gene sequences of interest.

Preliminarily, the above generalization of the procedure described in the preceding examples suggests that the inverse of the T4/T8 procedure may also be used. That is, the inventors consider that the amplifier immunomodulators of the °379 patent and °724 application are associated with T4 cells. Hence, T4 RNA material can be purified of the genetic material that is also associated with T8 cells, by reversing the roles of T4 and T8 in the foregoing procedure, thereby making available purified genetic material that is suitable for use in causing amplifiers to be manufactured. Such a T4 procedure may be effected by reversing the roles of T4 and T8 in the preceding examples.

The preceding generalization of the procedure also suggests extension of the T4/T8 procedure to human genetic materials other than those of T4 and T8 cells, such as Null Killer cells. It also suggests its extension to nonhuman genetic material, such as that of monkeys, cows, horses, dogs, etc., and to interspecies combinations. Further, it suggests a procedure beginning with DNA and utilizing cRNA, instead of RNA and cDNA, and as already suggested there need be no RNA/cDNA procedure based on use of reverse transcriptase but any procedure involving obtaining a complementary material, whether by synthesis or otherwise.

Example 10 --Preparation of Monkey Suppressor

A similar procedure is applied to the peripheral blood lymphocytes of a Rhesus monkey. T4 and T8 cells are identified and separated by flow cytometry, using the commercially available antibodies against human T4 and T8 cells (Ortho Pharm. Co.). These antibodies cross-react with T4 and T8 cells of Rhesus and other monkeys. See, e.g., Haynes, Dowell, Hensley, Gore, and Metzgar, Science 215, 298-99 (Jan. 15, 1982). The procedures carried out after isolation of the T4 or T8 cells are identical to those described above for human lymphocytes.

Example 11 --Preparation of Human Amplifier

The procedures of Examples 1-7 are repeated with the roles of T4 and T8 materials reversed. Then the procedure of Examples 8-9 is followed, but the resulting materials are amplifiers, rather than suppressors.

Multiple separations

While the T8 RNA extraction procedure described above contemplates two genetic materials, which contain three sets of gene sequences - (A + B + C, where A is T4-unique, B is T8-unique, and C is T4/T8-common), the procedure can be extended to three or more genetic materials. This is advantageous because T8 cells have more than suppressor activity, and T4 cells have more than amplifier activity. It is a substantial purification to remove T4/T8-common genetic material from an extract of T4 or T8, and greatly improves the yield of genetic manufacture of suppressors or amplifiers. However, the more extraneous activity that can be stripped from the genetic material, the better the yield of desired substances will be. Accordingly, a three-way separation that takes still more extraneous activity from the purified gene sequence product of the preceding procedure is advantageous. The inventors believe that T8 cells share some activities with Null Killer and B cells, and that T8 cells have some gene sequences that are common with them and are not common with T4 cells; therefore, if gene sequences common to T8 cells and either such cell are removed from the T8 gene product used for cloning, then the purified T8 gene product will be more specifically directed to uniquely T8-associated activities, including the suppressor activity of interest.

In such a procedure, the model of $A+B+C$, used previously, in the illustration at the beginning of the "Summary of the Present Invention," may appropriately be replaced by the model of $A+B+C+D$, where:

● T8 cells have gene sequences $A+B+C+D$;

● T8 cells share $B+C$ with T4 cells and share $C+D$ with T' cells, where T' is the third cell;

● T4 and T' cells both have $C$ sequences; and

● The T8 sequence of interest, because it is uniquely T8-associated, is $A$.

In addition, T4 and T' cells have other sequences not appreciably shared with T8 cells, and not of interest in this context. One separation on an affinity column allows the removal of $B+C$; another permits removal of $C+D$. The order of removal appears to be immaterial, and $C$ appears to be equally subtractable in either order of removal. The result of the procedure is to leave $A$ unaccompanied by $B+C+D$.

Example 12 --Three-way Separation

A separation of specially T8-associated gene sequences from gene sequences associated with T4 and T' cells is performed by, first, carrying out the procedure of Example 6, thereby producing a first separation product; and then, second, doing as follows:

The product of Example 5 is prepared with T' material instead of T4 material, thereby producing a further DNA-cellulose product. The procedure of Example 6 is carried out with (1) the first separation product instead of T8 mRNA, and (2) the further DNA-cellulose product instead of the DNA-cellulose product of original Example 5.

The resulting product is a mixture of substantially pure T8-associated mRNA and only very small amounts of RNA also found in T4 and T' cells.

Three is not an upper limit. The limit of the number of possible separations has as an absolute upper limit only the number of different appropriate genetic materials available. An appropriate genetic material is one: (1) that does not include the T8-associated gene sequences of interest, to a substantial extent; and (2) that does include, to a substantial extent, some gene sequences that are present in T8 cells and that are not of interest and are not included in T4 cells or other previously used subtraction materials. From an economic standpoint, the question of further losses in each separation and the additional labor must be considered. Also, the benefit of subtracting undesired genetic material reaches a point of diminishing returns.

In principle, however, the procedure may be extended to $i$ sets of gene sequences. That is, gene sequences uniquely associated with cell type $Gi$ may be separated from gene sequences that: - (1) naturally occur with the former in cell type $Gi$; and (2) are associated also with cell types G1, G2, G3, . . . $G(i-l)$. This separation can be accomplished by following these $(i-l)$ steps:

(1) The procedure of Example 5 is followed to prepare, first, a G1 DNA-cellulose product - (rather than the T4 DNA-cellulose product of original Example 5). Then, the procedure of Example 6 is followed with Gi mRNA (rather than the T8 mRNA of original Example 6).

(2) The procedure of Example 5 is followed to prepare, second, a G2 DNA-cellulose product - (rather than the T4 DNA-cellulose product of original Example 5). Then, the procedure of Example 6 is followed with the mRNA product produced by the preceding step (rather than the T8 mRNA of original Example 6).

(3) The procedure of Example 5 is followed to prepare, third, a G3 DNA-cellulose product - (rather than the T4 DNA-cellulose product of original Ex ample 5). Then, the procedure of Example 6 is followed with the mRNA product produced by the preceding, i.e., second, step (rather than the T8 mRNA of original Example 6).

(i-l) The procedure of Example 5 is followed to prepare, last, a G(i-l) DNA-cellulose product - (rather than the T4 DNA-cellulose product of original Example 5). Then, the procedure of Example 6 is followed with the mRNA product produced by the immediately preceding, i.e., (i-2)th, step (rather than the T8 mRNA of original Example 6).

In the resulting product, the gene sequences that cell type $G_i$ shares with any of cell types G1, G2, G3, . . . $G(i-1)$ are substantially all removed and the gene sequences remaining in the final product are substantially only those sequences that cell type $G_i$ shares with none of them.

## GENERAL CONCLUDING REMARKS

The above described procedures disclose what the inventors believe is a unique and hitherto unknown method of extracting relatively pure gene sequences from materials derived from living human beings and animals, thereby making the gene sequences available for cloning purposes. Although the procedure was described primarily in terms of extracting human T8 gene sequences useful for genetically engineered manufacture of suppressor material, the inventors do not consider the invention to be limited to that purpose. The novel technique disclosed herein is adaptable, by means known to those skilled in the art, to the extraction of other human genetic material and similar mammalian or other animal, natural or synthetic, genetic material. The scope of the invention extends also to the materials so extracted, which have hitherto been unavailable in a form substantially purified from extraneous genetic material, and to the use of the novel purified material in genetic manufacture of products whose cellular generation is associated with the presence of the gene sequences that have been provided by the procedure described herein.

While the invention has been described primarily in connection with a specific and preferred embodiment thereof, it will be understood that it is capable of further modifications without departing from the spirit and scope of the invention. This application is intended to cover all variations, uses, or adaptations of the invention, following, in general, the principles of the invention and including such departures from the present disclosure as come within known or customary practice within the art to which the invention pertains, or as are obvious to persons skilled in the art, at the time the departure is made.

Terminology of claims

As used in the following claims, the term **nucleic acid** is used generically to include DNA and RNA. The term **genetic material** refers to a mixture of material in which there is contained nucleic acid material. The term **denatured** refers to the dissociation of double stranded nucleic acid to single strands. The term **cut up** refers to the fractionation of nucleic acid into segments or fragments. The segments or fragments contain **gene sequences** or **nucleic acid sequences.**

The term **support** refers to resins, such as activated cellulose resin, microspheres, silica, and other materials suitable for binding nucleic acid or fragments thereof. Such supports have a number of **binding sites** on them to which nucleic acid or fragments thereof can be bound. If all of the binding sites are already occupied by nucleic acid or fragments thereof, no further nucleic acid or fragments can be bound to the support.

The terms **enriched** and **impoverished** are used to refer to the gene sequence content of purified genetic material. The terms are used, respectively, to refer to increasing and decreasing the relative frequency or content of the gene sequences in question. Thus, when the nucleic acid of T4 cells (as found in nature) contains gene sequences $\underline{A}$ and $\underline{B}$, and a method is used whereby a product is provided containing more of $\underline{A}$ and less of $\underline{B}$ than in nature, it may be said (in the terminology used here) that the resulting product is enriched in $\underline{A}$ and impoverished in $\underline{B}$.

As has been taught above, an object of this invention is to produce a purified product from T8 cells in which product the content of gene sequences associated with both T4 and T8 cells is impoverished and the content of gene sequences associated only with T8 cells is enriched. It is not necessarily the case, however, that the process will remove virtually all of the former matierial and leave virtually nothing but the latter material. That is a goal, but it is not necessarily fully achieved. By the same token, the purification contemplated herein is not necessarily total; it is a relative concept.

The term **associated with** is used to refer to gene sequences that are said to be associated with the in vivo production of nongenetic material. The nongenetic material manufactured in accordance with the teachings of this invention may be a protein coded for by the genetic material of the invention. The nongenetic material may be a biochemical whose manufacture by a cell is in some way regulated by a protein coded for by the genetic material of the invention. One example of this association is that of the genetic material of T8 cells, which may be used to manufacture suppressors, which regulate the operation of the immune system. Such genetic material is associated with the in vivo production of suppressors. However, as explained above, the invention is not limited to that particular species of the invention, but extends more generically to the genera within which that species falls, as claimed below.

When it is said that the materials are brought into contact "under hybridizing conditions," it is meant to import the complementarity of the two materials that are to hybridize, as well as the conventional temperature, pH, and other conditions requisite to hybridization. Therefore, the term "under hybridizing conditions" should be understood to mean that the materials referred to are complementary, such as DNA and RNA.

The term "selected" is used at times to refer to sequences and cell types, to emphasize that the claimed product is the result of a deliberate purification process and not an accident. When the phrase "unaccompanied by more than an insubstantial amount of a selected second set of gene sequences" is used to describe the properties of a claimed product, the phrase means that in the claimed product there is less than 10% of the gene sequences sought to be excluded.

At times the limitation is added that "essentially all" gene sequences from a second cell are excluded. In this context, it is meant that at least 99% are excluded within the limits of present measuring techniques. That is, the procedure of Examples 1-6, when carried out under optimal conditions, is believed to remove all of the unwanted sequences that it is possible to measure, other than a minimal trace amount.

## Claims

1. A method of obtaining from a first genetic material a product that is enriched in nucleic acid sequences that are contained in said first genetic material and that is impoverished in nucleic acid sequences that are contained in a second genetic material, said method comprising the following steps:

(1) Providing said first genetic material in denatured form;

(2) Providing fragments of denatured nucleic acid of said second genetic material, said fragments being bound to a support;

(3) Bringing into contact with one another, under hybridizing conditions, said denatured first genetic material and said denatured fragments bound to said support; and

(4) Collecting said denatured first genetic material that has not become bound to said denatured fragments bound to said support, whereby said product is provided.

2. The method of claim 1 wherein at least one of said materials consists essentially of animal genetic materials.

3. The method of claim 2 wherein at least one of said materials consists essentially of mammalian genetic materials.

4. The method of claim 3 wherein at least one of said materials consists essentially of human genetic materials.

5. The method of claim 1 wherein said support is a resin.

6. The method of claim 5 wherein said resin is activated cellulose resin.

7. The method of claim 1 wherein said first genetic material is an RNA and said second genetic material is a cDNA.

8. The method of claim 7 wherein said second genetic material is a cDNA obtained by reverse transcription from a second RNA.

9. The method of claim 7 wherein said first genetic material is derived from T8 cells, and said second genetic material is derived from T4 cells.

10. The method of claim 7 wherein said first genetic material is derived from T4 cells, and said second genetic material is derived from T8 cells.

11. The method of claim 1 wherein said fragments are at least approximately 500 base pairs.

12. The method of claim 11 wherein said fragments are approximately 500 to 5000 base pairs.

13. The method of claim 1 wherein said denatured fragments are provided to said support in excess of the amount necessary to occupy all nucleic acid binding sites of said support, whereby said support has essentially no unoccupied said binding sites when said step (3) occurs.

14. A method of obtaining, from human RNA material derived from T8 cells, an RNA product that consists essentially of gene sequences contained in T8 cells and in whch are uncommon any gene sequences contained in T4 cells, said method comprising the following steps:

(1) Providing a T4 RNA material consisting essentially of RNA from T4 cells;

(2) Preparing by reverse transcription from said T4 RNA material a T4 cDNA material consisting essentially of genetic material that is complementary to said T4 RNA material;

(3) Providing said T4 cDNA material in denatured and cut up form, and binding it to a resin;

(4) Providing in denatured form said human RNA material derived from T8 cells;

(5) Bringing into contact with one another in an affinity column said materials of steps (3) and (4); and

(6) Collecting a wash-through from the column,

whereby said RNA product is provided.

15. A method of obtaining, from a first genetic material that includes a first group of gene sequences and also includes a plurality of other gene sequences that are respectively contained in a plurality of other genetic materials, a gene product that is enriched in said first group of gene sequences and that is impoverished in said other gene sequences, said method comprising the following steps:

(1) Providing said first genetic material in denatured form;

(2) Providing fragments of denatured nucleic acid of one of said plurality of other genetic materials, said fragments being bound to a support:

(3) Bringing into contact with one another, under hybridizing conditions, said denatured first genetic material and said denatured fragments bound to said support; and

(4) Collecting said denatured first genetic material that has not become bound to said denatured fragments bound to said support, whereby an extract is obtained;

(5) Repeating at least one further time said series of steps (2) through (4), wherein each said further time that said series of steps is repeated:

(a) further fragments of denatured nucleic acid of another one of said plurality of other genetic materials are provided in the reiterated version of step (2), said further fragments being bound to a further support,

(b) said extract of the immediately preceding version of step (4) is brought into contact with said further fragments in the reiterated version of step - (3), and

(c) a further extract is collected in the reiterated version of step (4),

whereby said product is provided as the extract of the final step of said method.

16. Purified genetic material that essentially consists of a selected first set of gene sequences unaccompanied by more than an insubstantial amount of a selected second set of gene sequences, where said first set of gene sequences naturally occurs in the nucleic acid of a selected first type of cell accompanied by said second set of gene sequences, where said second set of gene sequences naturally occurs also in the nucleic acid of a selected second type of cell, and where at least one of said types of cell is animal.

17. Purified genetic material that essentially consists of a first set of gene sequences from the nucleic acid of a first type of cell, where said genetic material has been purified to exclude therefrom at least some of at least one second set of gene sequences that naturally occur in the nucleic acid of both said first type of cell and a second type of cell, and where at least one of said types of cell is animal.

18. Purified genetic material of claim 17 from which has been excluded essentially all sets of gene sequences that naturally occur in said second type of cell.

19. Purified genetic material of claim 18 wherein said cells are human T cells.

20. Material of claim 19 wherein said first type of cell is T8 and said second type of cell is T4.

21. Material of claim 19 wherein said first type of cell is T4 and said second type of cell is T8.

22. Material of claim 19 impoverished of gene sequences that naturally occur in the nucleic acid of least one non-T8 lymphocyte cell.

23. A plasmid containing an insert of material of claim 19.

24. E. coli that have been transformed by plasmids of claim 23.

25. E. coli of claim 24 wherein said inserts are of a predetermined size.

26. E. coli of claim 25 wherein said inserts are approximately 500 to 5000 base pairs.

27. Material of claim 17 wherein said material and cells are those of a mammal.

28. A plasmid containing an insert of material of claim 27.

29. E. coli that have been transformed by plasmids of claim 28.

30. E. coli of claim 29 wherein said inserts are of a predetermined size.

31. E. coli of claim 30 wherein said inserts are approximately 500 to 5000 base pairs.

32. A method of using genetic material of claim 17 to manufacture biologically active nongenetic material, said method comprising the following steps:

(1) Cloning said genetic material into a host bacterium, where said genetic material includes gene sequences from cells associated with the in vivo production of said biologically active nongenetic material;

(2) Culturing said host bacterium, thereby producing a culture; and

(3) Extracting said biologically active nongenetic material from said culture.

33. The method of claim 32 wherein said genetic material is derived from human T cells.

34. The method of claim 32 wherein said nongenetic material is a human immunomodulator.

35. The method of claim 32 wherein said genetic material is derived from mammalian cells.

36. The method of claim 32 wherein said nongenetic material is a mammalian immunomodulator.

37. A method of manufacturing human immunomodulators that comprises the following steps:

(1) Cloning purified genetic material of claim 19 into E. coli;

(2) Culturing said E. coli, thereby producing a culture;

(3) Harvesting said culture; and

(4) Extracting immunomodulators from said culture.

38. The method of claim 37 wherein the immunomodulator is a suppressor, the purified genetic material is derived from T8 cells, and said material has been impoverished in genetic material derived from T4 cells.

## DOCUMENTS CONSIDERED TO BE RELEVANT

EP 86110000.6

| Category | Citation of document with indication, where appropriate, of relevant passages | Relevant to claim | CLASSIFICATION OF THE APPLICATION (Int. Cl.4) |
|---|---|---|---|
| P,A | <u>EP - A1 - 0 157 235</u> (BAYER AG)<br>* Claim 1 *<br>-- | 1 | C 12 N 15/00<br>C 12 P 21/00<br>C 12 N  1/20<br>//( C 12 N 15/00<br>C 12 R  1:19) |
| A | <u>EP - A2 - 0 072 925</u> (RUTGERS RE-SEARCH AND EDUCATIONAL FOUNDATION)<br>* Claims 1,2 *<br>-- | 1,9,<br>10,17,<br>20,21 | |
| D,A | <u>US - A - 4 468 379</u> (GOTTLIEB)<br>* Claims 19,20 *<br>---- | 32,34,<br>37,38 | |

| | |
|---|---|
| | **TECHNICAL FIELDS SEARCHED (Int. Cl.4)** |
| | C 12 N<br>C 12 P<br>A 61 K |

The present search report has been drawn up for all claims

| Place of search | Date of completion of the search | Examiner |
|---|---|---|
| VIENNA | 23-10-1986 | WOLF |

EPO Form 1503 03 82